# EUROPEAN PATENT SPECIFICATION

(11) **EP 3 429 522 B1**
(45) Date of publication and mention of the grant of the patent: **03.09.2025**
(21) Application number: 17715527.2
(22) Date of filing: 13.03.2017
(51) Int. Cl.: A61M 1/00, A61F 13/05, A61F 13/00

(54) **WOUND DRESSING APPARATUS WITH FLEXIBLE DISPLAY**
WUNDVERBANDVORRICHTUNG MIT FLEXIBLER ANZEIGE
APPAREIL DE PANSEMENT DE PLAIE À AFFICHAGE SOUPLE

(30) Priority: 14.03.2016 US 201662307790 P
(43) Date of publication of application: 23.01.2019
(73) Proprietor: Smith & Nephew plc, Watford, Hertfordshire WD18 8YE (GB)
(72) Inventor: QUINTANAR, Felix C., Hull, HU3 2BN (GB); HARTWELL, Edward Yerbury, Hull, HU3 2BN (GB)
(74) Representative: Guy, Mark Robert
(86) International application number: PCT/IB2017/000339
(87) International publication number: WO 2017/158428

(56) References cited:
- WO-A1-2012/141999
- US-A1- 2012 330 252
- US-A1- 2013 123 722
- US-A1- 2015 174 304

## Description

### CROSS-REFERENCE TO RELATED APPLICATIONS

This application claims priority to U.S. Provisional Application No. 62/307,790, filed March 14, 2016.

### BACKGROUND

### Technical Field

Embodiments described herein relate to apparatuses used in combination with negative pressure wound therapy.

### Description of the Related Art

The treatment of open or chronic wounds that are too large to spontaneously close or otherwise fail to heal by means of applying negative pressure to the site of the wound is well known in the art. Negative pressure wound therapy (NPWT) systems currently known in the art commonly involve placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound site and assisting the body's normal inflammatory process while simultaneously removing excess fluid, which may contain adverse cytokines or bacteria. However, further improvements in NPWT are needed to fully realize the benefits of treatment.

Many different types of wound dressings are known for aiding in wound healing. These different types of wound dressings include many different types of materials and layers, for example, gauze, pads, foam pads or multi-layer wound dressings. One example of a multi-layer wound dressing is the PICO dressing, available from Smith & Nephew, which includes a superabsorbent layer beneath a backing layer to provide a canister-less system for treating a wound with NPWT. The wound dressing may be sealed to a suction port providing connection to a length of tubing, which may be used to pump fluid out of the dressing or to transmit negative pressure from a pump to the wound dressing. Another example of a multi-layer wound dressing is the ALLEVYN Life dressing, available from Smith & Nephew, which includes a moist wound environment dressing that is used to treat the wound without the use of negative pressure.

Wound dressings for use in treating wounds do not allow visualization of the wound site during healing. The clinician or user is required to at least partially remove the dressing to gain access to the wound area and assess healing or complications, it may be desirable, in some situations, to provide a wound dressing that allows visualization of the wound in real time to assess the condition of the wound. US2013/123722A1 discloses wound dressings, systems, and methods for treating a wound on a patient's limb, such as a venous leg ulcer. The dressings, systems, and methods involve creating airflow within the dressing to vaporize and remove liquid. US2012/330252A1 discloses a reduced-pressure medical treatment system for treating a tissue site on a patient that includes a medical drape that has an holographically-formed polymer dispersed liquid crystal (H-PDLC) device that changes visual appearance when experiencing strain. US2015/174304A1 discloses embodiments having a pump assembly mounted to or supported by a dressing for reduced pressure wound therapy. The dressing can have visual pressure, saturation, and/or temperature sensors to provide a visual indication of the level of pressure, saturation, and/or temperature within the dressing.

### SUMMARY

Embodiments of the present disclosure relate to apparatuses and methods for wound treatment. Some of the wound treatment apparatuses described herein comprise a negative pressure source or a pump assembly or system for providing negative pressure to a wound. Wound treatment apparatuses may also comprise wound dressings that may be used in combination with the negative pressure sources and pump assemblies described herein. The invention, relating to a negative pressure wound therapy apparatus, is defined by the appended claims. Embodiments of the present disclosure that fall outside the scope of the appended claims, for example systems and methods for wound treatment, are provided for understanding of the invention only.

In aspects of the disclosure according to the present invention, a negative pressure wound therapy apparatus is provided as defined by claim 1 or claim 2.

The apparatus of the invention may also include any combination of the following features described in this paragraph, among others described herein. In some embodiments, the apparatus can further include the one or more sensors configured to be positioned on or within the wound dressing, the one or more sensors configured to provide information about the condition of the wound. In some embodiments, the apparatus can further include the controller positioned within or on the wound dressing. In some embodiments, the apparatus can further include the controller configured to be positioned remote from the wound dressing. In some embodiments, the controller can be configured to process the information provided by the sensors and display the information on the dressing display. In some embodiments, the dressing display can be a flexible organic light emitting diode display or e-ink display. In some embodiments, the sensors can be configured to measure parameters such as temperature, pH, oxygen, carbon dioxide, conductivity, inductance, lactate, metallomatric proteases, growth factors, optical absorption and reflectance, or infection. In some embodiments, the dressing display can be configured to display an image of the wound. In some embodiments, the apparatus can further include a negative pressure source configured to apply negative pressure to the wound, in some embodiments, the apparatus can further include a wound contact layer configured to be positioned in contact with the wound. In some embodiments, the apparatus can further include one or more superabsorbent layers positioned between the wound contact layer and the backing layer. In some embodiments, the apparatus can further include a spacer layer between the wound contact layer and the backing layer.

Any of the features, components, or details of any of the arrangements or embodiments disclosed in this application, including without limitation any of the pump embodiments and any of the negative pressure wound therapy embodiments disclosed below, are interchangeably combinable with any other features, components, or details of any of the arrangements or embodiments disclosed herein to form new arrangements and embodiments.

### BRIEF DESCRIPTION OF THE DRAWINGS

Figure 1A illustrates an embodiment not according to the present invention of a negative pressure wound treatment system employing a flexible fluidic connector and a wound dressing capable of absorbing and storing wound exudate;
Figure 1B illustrates an embodiment not according to the present invention of a negative pressure wound treatment system employing a flexible fluidic connector and a wound dressing capable of absorbing and storing wound exudate;
Figure 2A illustrates an embodiment not according to the present invention of a negative pressure wound treatment system employing a flexible fluidic connector and a wound dressing capable of absorbing and storing wound exudate;
Figure 2B illustrates a cross section of an embodiment not according to the present invention of a fluidic connector connected to a wound dressing;
Figure 3 illustrates an embodiment not according to the present invention of a wound treatment system employing a wound dressing capable of absorbing and storing wound exudate to be used without negative pressure;
Figures 4A-D illustrate the use and application of an embodiment not according to the present invention of a wound treatment system onto a patient;
Figure 5 illustrates an embodiment of a wound dressing according to the present invention with a dressing display on the wound dressing; and
Figures 6A and 6B illustrate a schematic illustration of a wound that can be displayed on the dressing display.

### DETAILED DESCRIPTION

Embodiments disclosed herein relate to apparatuses and methods of treating a wound with or without reduced pressure, including optionally a source of negative pressure and wound dressing components and apparatuses. The apparatuses and components comprising the wound overlay and packing materials, if any, are sometimes collectively referred to herein as dressings. In some embodiments, the wound dressing can be provided to be utilized without reduced pressure.

Preferred embodiments disclosed herein relate to wound therapy for a human or animal body. Therefore, any reference to a wound herein can refer to a wound on a human or animal body, and any reference to a body herein can refer to a human or animal body. The term "wound" as used herein, in addition to having its broad ordinary meaning, includes any body part of a patient that may be treated using negative pressure. It is to be understood that the term wound is to be broadly construed and encompasses open and closed wounds in which skin is torn, cut or punctured or where trauma causes a contusion, or any other superficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sternotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

Treatment of such wounds can be performed using negative pressure wound therapy, wherein a reduced or negative pressure can be applied to the wound to facilitate and promote healing of the wound. It will also be appreciated that the wound dressing and methods as disclosed herein may be applied to other parts of the body, and are not necessarily limited to treatment of wounds.

It will be understood that embodiments of the present disclosure are generally applicable to use in topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g.,-40 mmHg is less than -60 mmHg). Negative pressure that is "more" or "greater" than -X mmHg corresponds to pressure that is further from atmospheric pressure (e.g., -80 mmHg is more than -60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, or in synchronization with one or more patient physiological indices (e.g., heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010.

Embodiments of the wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," U.S. Patent Application No. 14/418874, filed January 30, 2015, published as US 2015/0190286 A1 on July 9, 2015, titled "WOUND DRESSING AND METHOD OF TREATMENT,". Embodiments of the wound dressings, wound treatment apparatuses and methods described herein may also be used in combination or in addition to those described in U.S. Patent Application No. 13/092,042, filed April 21 2011, US Patent No. 9,061,095, titled "WOUND DRESSING AND METHOD OF USE," and U.S. Patent Application No. 14/715,527, filed May 18, 2015, titled "FLUIDIC CONNECTOR FOR NEGATIVE PRESSURE WOUND THERAPY,", including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Patent Application No. PCT/EP2016/059329, filed on April 26, 2016, entitled "REDUCED PRESSURE APPARATUSES", published as WO 2016/174048, on November 3, 2016. In some of these embodiments, the pump or associate electronic components may be integrated into the wound dressing to provide a single article to be applied to the wound.

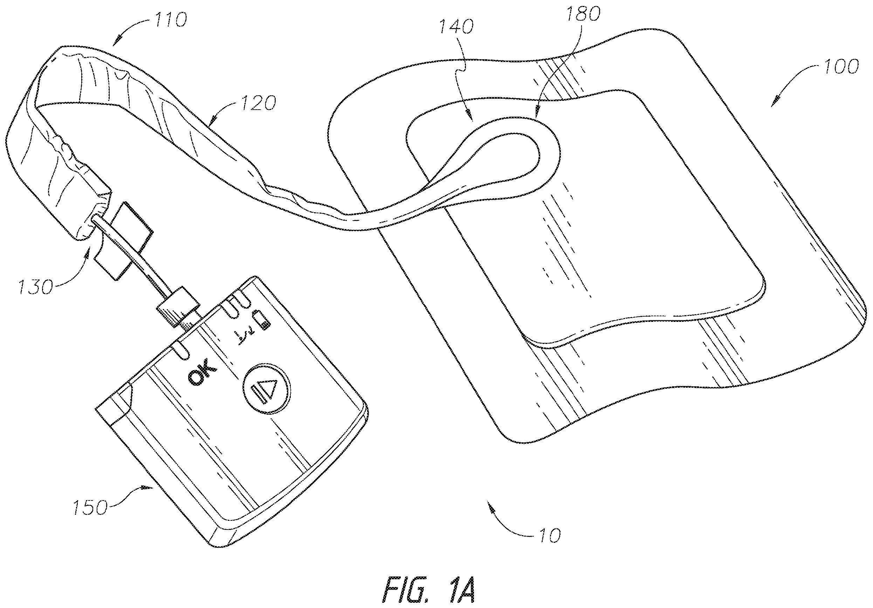

Figures 1A-B illustrate embodiments of a negative pressure wound treatment system 10 employing a wound dressing 100 in conjunction with a fluidic connector 110. Here, the fluidic connector 110 may comprise an elongate conduit, more preferably a bridge 120 having a proximal end 130 and a distal end 140, and an applicator 180 at the distal end 140 of the bridge 120. An optional coupling 160 is preferably disposed at the proximal end 130 of the bridge 120. A cap 170 may be provided with the system (and can in some cases, as illustrated, be attached to the coupling 160). The cap 170 can be useful in preventing fluids from leaking out of the proximal end 130. The system 10 may include a source of negative pressure such as a pump or negative pressure unit 150 capable of supplying negative pressure. The pump may comprise a canister or other container for the storage of wound exudates and other fluids that may be removed from the wound. A canister or container may also be provided separate from the pump. In some embodiments, such as illustrated in Figures 1A-1B, the pump 150 can be a canisterless pump such as the PICO^{™} pump, as sold by Smith & Nephew. The pump 150 may be connected to the coupling 160 via a tube 190, or the pump 150 may be connected directly to the coupling 160 or directly to the bridge 120. in use, the dressing 100 is placed over a suitably-prepared wound, which may in some cases be filled with a wound packing material such as foam or gauze. The applicator 180 of the fluidic connector 110 has a sealing surface that is placed over an aperture in the dressing 100 and is sealed to the top surface of the dressing 100. Either before, during, or after connection of the fluidic connector 110 to the dressing 100, the pump 150 is connected via the tube 190 to the coupling 160, or is connected directly to the coupling 160 or to the bridge 120. The pump is then activated, thereby supplying negative pressure to the wound. Application of negative pressure may be applied until a desired level of healing of the wound is achieved.

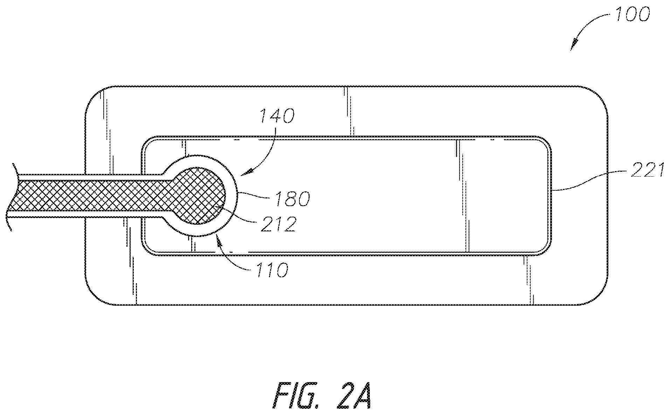

As shown in Figure 2A, the fluidic connector 110 preferably comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 10 may comprise two or more fluidic connectors 110, each comprising one or more heads 140, in fluidic communication therewith. In a preferred embodiment, the head 140 may measure 30mm along its widest edge. The head 140 forms at least in part the applicator 180, described above, that is configured to seal against a top surface of the wound dressing.

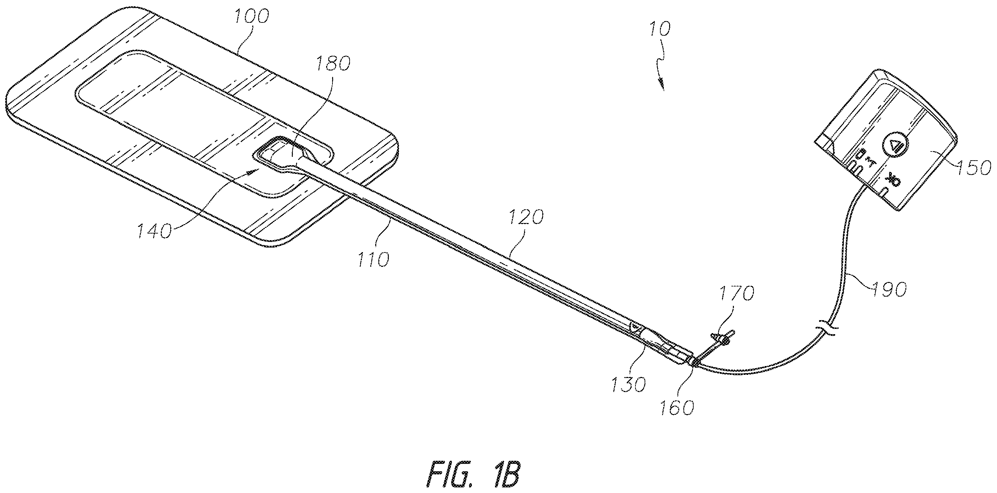

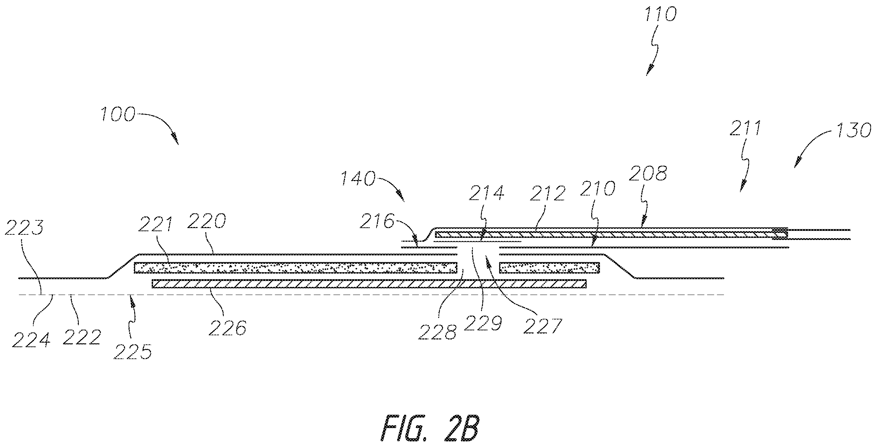

Figure 2B illustrates a cross-section through a wound dressing 100 similar to the wound dressing 10 as shown in Figure 1B and described in International Patent Publication WO2013175306 A2, along with fluidic connector 110. The wound dressing 100, which can alternatively be any wound dressing embodiment disclosed herein or any combination of features of any number of wound dressing embodiments disclosed herein, can be located over a wound site to be treated. The dressing 100 may be placed as to form a sealed cavity over the wound site. In a preferred embodiment, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Figure 2B, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 222 may help maintain the integrity of the entire dressing 100 while also creating an air tight seal around the absorbent pad in order to maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A layer 226 of porous material can be located above the wound contact layer 222. This porous layer, or transmission layer, 226 allows transmission of fluid including liquid and gas away from a wound site into upper layers of the wound dressing. In particular, the transmission layer 226 preferably ensures that an open air channel can be maintained to communicate negative pressure over the wound area even when the absorbent layer has absorbed substantial amounts of exudates. The layer 226 should preferably remain open under the typical pressures that will be applied during negative pressure wound therapy as described above, so that the whole wound site sees an equalized negative pressure. The layer 226 may be formed of a material having a three dimensional structure. For example, a knitted or woven spacer fabric (for example Baltex 7970 weft knitted polyester) or a non-woven fabric could be used.

In some embodiments, the transmission layer 226 comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 10 denier flat polyester and a third layer formed sandwiched between these two layers which is a region defined by a knitted polyester viscose, cellulose or the like monofilament fiber. Other materials and other linear mass densities of fiber could of course be used.

Whilst reference is made throughout this disclosure to a monofilament fiber it will be appreciated that a multistrand alternative could of course be utilized. The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer 221 helps lock the liquid away or itself wicks the liquid onwards towards the cover layer where it can be transpired.

Preferably, to improve the liquid flow across the transmission layer 226 (that is to say perpendicular to the channel region formed between the top and bottom spacer layers, the 3D fabric may be treated with a dry cleaning agent (such as, but not limited to, Perchloro Ethylene) to help remove any manufacturing products such as mineral oils, fats or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

A layer 221 of absorbent material is provided above the transmission layer 226. The absorbent material, which comprise a foam or non-woven natural or synthetic material, and which may optionally comprise a super-absorbent material, forms a reservoir for fluid, particularly liquid, removed from the wound site. In some embodiments, the layer 10 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 221 may typically be manufactured from ALLEVYN^{™} foam, Freudenberg 114-224-4 or Chem-Posite^{™}11C-450. In some embodiments, the absorbent layer 221 may comprise a composite comprising superabsorbent powder, fibrous material such as cellulose, and bonding fibers. In a preferred embodiment, the composite is an airlaid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is preferably provided in the backing layer 220 to allow a negative pressure to be applied to the dressing 100. The fluidic connector 110 is preferably attached or sealed to the top of the backing layer 220 over the orifice 227 made into the dressing 100, and communicates negative pressure through the orifice 227. A length of tubing may be coupled at a first end to the fluidic connector 110 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Preferably the absorbent layer 221 includes at least one through hole 228 located so as to underlie the fluidic connector 110. The through hole 228 may in some embodiments be the same size as the opening 227 in the backing layer, or may be bigger or smaller. As illustrated in Figure 2B a single through hole can be used to produce an opening underlying the fluidic connector 110. It will be appreciated that multiple openings could alternatively be utilized. Additionally should more than one port be utilized according to certain embodiments of the present disclosure one or multiple openings may be made in the absorbent layer and the obscuring layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in Figure 2B. This allows the negative pressure applied to the fluidic connector 110 to be communicated to the transmission layer 226 without passing through the absorbent layer 221. This ensures that the negative pressure applied to the wound site is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described in international Patent Publication WO2014/020440, may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the backing layer 220 and a wound site where a negative pressure can be established. The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments the moisture vapor permeability of the backing layer increases when the backing layer becomes wet. The moisture vapor permeability of the wet backing layer may be up to about ten times more than the moisture vapor permeability of the dry backing layer.

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in Figures 2A-2B, the absorbent layer 221 may define a smaller perimeter than that of the backing layer 220, such that a boundary or border region is defined between the edge of the absorbent layer 221 and the edge of the backing layer 220.

As shown in Figure 2B, one embodiment of the wound dressing 100 comprises an aperture 228 in the absorbent layer 221 situated underneath the fluidic connector 110. In use, for example when negative pressure is applied to the dressing 100, a wound facing portion of the fluidic connector may thus come into contact with the transmission layer 226, which can thus aid in transmitting negative pressure to the wound site even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 11, or the orifice 227.

In particular for embodiments with a single fluidic connector 110 and through hole, it may be preferable for the fluidic connector 110 and through hole to be located in an off-center position as illustrated in Figure 2A. Such a location may permit the dressing 100 to be positioned onto a patient such that the fluidic connector 110 is raised in relation to the remainder of the dressing 100. So positioned, the fluidic connector 110 and the filter 214 may be less likely to come into contact with wound fluids that could prematurely occlude the filter 214 so as to impair the transmission of negative pressure to the wound site.

Turning now to the fluidic connector 110, preferred embodiments comprise a sealing surface 216, a bridge 211 (corresponding to bridge 120 in Figures 1A-1B) with a proximal end 130 and a distal end 140, and a filter 214. The sealing surface 216 preferably forms the applicator previously described that is sealed to the top surface of the wound dressing. In some embodiments a bottom layer of the fluidic connector 110 may comprise the sealing surface 216. The fluidic connector 110 may further comprise an upper surface vertically spaced from the sealing surface 216, which in some embodiments is defined by a separate upper layer of the fluidic connector. In other embodiments the upper surface and the lower surface may be formed from the same piece of material. In some embodiments the sealing surface 216 may comprise at least one aperture 229 therein to communicate with the wound dressing. In some embodiments the filter 214 may be positioned across the opening 229 in the sealing surface, and may span the entire opening 229. The sealing surface 216 may be configured for sealing the fluidic connector to the cover layer of the wound dressing, and may comprise an adhesive or weld. In some embodiments, the sealing surface 216 may be placed over an orifice in the cover layer. In other embodiments, the sealing surface 216 may be positioned over an orifice in the cover layer and an aperture in the absorbent layer 221, permitting the fluidic connector 110 to provide air flow through the transmission layer 226. In some embodiments, the bridge 211 may comprise a first fluid passage 212 in communication with a source of negative pressure, the first fluid passage 212 comprising a porous material, such as a 3D knitted material, which may be the same or different than the porous layer 226 described previously. The bridge 211 is preferably encapsulated by at least one flexible film layer 208, 210 having a proximal and distal end and configured to surround the first fluid passage 212, the distal end of the flexible film being connected the sealing surface 216. The filter 214 is configured to substantially prevent wound exudate from entering the bridge.

Some embodiments may further comprise an optional second fluid passage positioned above the first fluid passage 212. For example, some embodiments may provide for an air leak may be disposed at the proximal end of the top layer that is configured to provide an air path into the first fluid passage 212 and dressing 100 similar to the suction adapter as described in U.S. Patent No 8,801,685.

Preferably, the fluid passage 212 is constructed from a compliant material that is flexible and that also permits fluid to pass through it if the spacer is kinked or folded over. Suitable materials for the fluid passage 212 include without limitation foams, including open-cell foams such as polyethylene or polyurethane foam, meshes, 3D knitted fabrics, non-woven materials, and fluid channels. In some embodiments, the fluid passage 212 may be constructed from materials similar to those described above in relation to the transmission layer 226. Advantageously, such materials used in the fluid passage 212 not only permit greater patient comfort, but may also provide greater kink resistance, such that the fluid passage 212 is still able to transfer fluid from the wound toward the source of negative pressure while being kinked or bent.

In some embodiments, the fluid passage 212 may be comprised of a wicking fabric, for example a knitted or woven spacer fabric (such as a knitted polyester 3D fabric, Baltex 7970^{®}, or Gehring 879^{®}) or a nonwoven fabric. These materials selected are preferably suited to channeling wound exudate away from the wound and for transmitting negative pressure or vented air to the wound site, and may also confer a degree of kinking or occlusion resistance to the fluid passage 212. In some embodiments, the wicking fabric may have a three-dimensional structure, which in some cases may aid in wicking fluid or transmitting negative pressure. In certain embodiments, including wicking fabrics, these materials remain open and capable of communicating negative pressure to a wound area under the typical pressures used in negative pressure therapy, for example between 40 to 150 miTiHg. In some embodiments, the wicking fabric may comprise several layers of material stacked or layered over each other, which may in some cases be useful in preventing the fluid passage 212 from collapsing under the application of negative pressure. In other embodiments, the wicking fabric used in the fluid passage 212 may be between 1.5 mm and 6 mm; more preferably, the wicking fabric may be between 3 mm and 6 mm thick, and may be comprised of either one or several individual layers of wicking fabric. In other embodiments, the fluid passage 212 may be between 1.2-3 mm thick, and preferably thicker than 1.5 mm. Some embodiments, for example a suction adapter used with a dressing which retains liquid such as wound exudate, may employ hydrophobic layers in the fluid passage 212, and only gases may travel through the fluid passage 212. Additionally, and as described previously, the materials used in the system are preferably conformable and soft, which may help to avoid pressure ulcers and other complications which may result from a wound treatment system being pressed against the skin of a patient.

Preferably, the filter element 214 is impermeable to liquids, but permeable to gases, and is provided to act as a liquid barrier and to ensure that no liquids are able to escape from the wound dressing 100. The filter element 214 may also function as a bacterial barrier. Typically the pore size is 0.2µm. Suitable materials for the filter material of the filter element 214 include 0.2 micron Gore^{™} expanded PTFE from the MMT range, PALL Versapore^{™} 200R, and Donaldson^{™} TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter element can be attached or sealed to the port or the cover film over the orifice. For example, the filter element 214 may be molded into the fluidic connector 110, or may be adhered to one or both of the top of the cover layer and bottom of the suction adapter 110 using an adhesive such as, but not limited to, a UV cured adhesive.

It will be understood that other types of material could be used for the filter element 214. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. In some embodiments, filter element 214 comprises a support layer and an acrylic co-polymer membrane formed on the support layer. Preferably the wound dressing 100 according to certain embodiments uses microporous hydrophobic membranes (MHMs). Numerous polymers may be employed to form MHMs. For example, the MHMs may be formed from one or more of PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter element 214 may also include an odor absorbent material, for example activated charcoal, carbon fiber cloth or Vitec Carbotec-RT Q2003073 foam, or the like. For example, an odor absorbent material may form a layer of the filter element 214 or may be sandwiched between microporous hydrophobic membranes within the filter element. The filter element 214 thus enables gas to be exhausted through the orifice. Liquid, particulates and pathogens however are contained in the dressing.

Similar to the embodiments of wound dressings described above, some wound dressings comprise a perforated wound contact layer with silicone adhesive on the skin-contact face and acrylic adhesive on the reverse. Above this bordered layer sits a transmission layer or a 3D spacer fabric pad. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

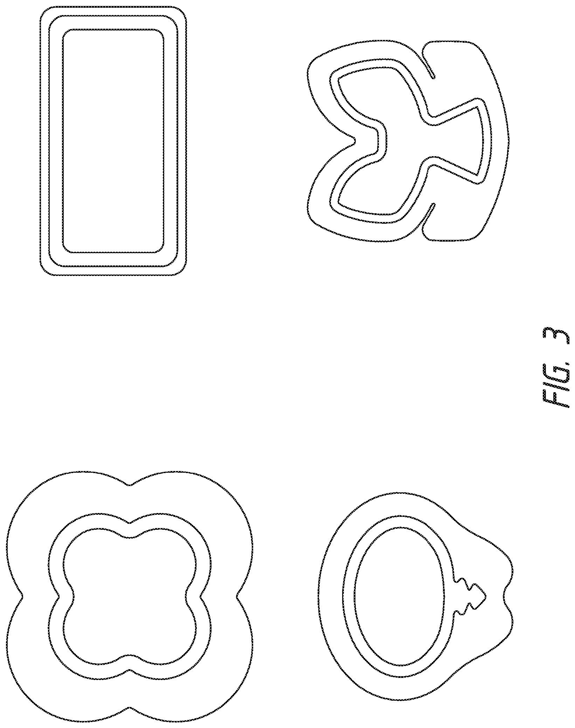

Figure 3 illustrates various embodiments of a wound dressing that can be used for healing a wound without negative pressure. As shown in the dressings of Figure 3, the wound dressings can have multiple layers similar to the dressings described with reference to Figures lA-B and 2A-B except the dressings of Figure 3 do not include a port or fluidic connector. The wound dressings of Figure 3 can include a cover layer and wound contact layer as described herein. The wound dressing can include various layers positioned between the wound contact layer and cover layer. For example, the dressing can include one or more absorbent layers or one or more transmission layers as described herein with reference to Figures 1A-B and 2A-B. Additionally, some embodiments related to wound treatment comprising a wound dressing described herein may also be used in combination or in addition to those described in U.S. Application Publication No. 2014/0249495, filed May 21, 2014, entitled "WOUND DRESSING AND METHOD OF TREATMENT", including further details relating to embodiments of wound dressings, the wound dressing components and principles, and the materials used for the wound dressings.

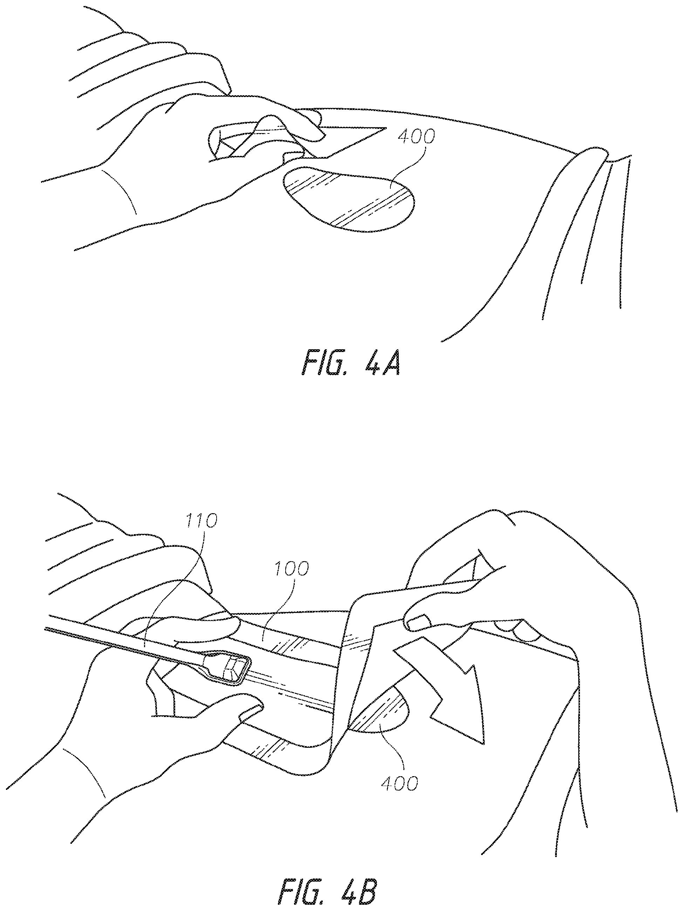

Figures 4A-D illustrate the use of an embodiment of a negative pressure therapy wound treatment system being used to treat a wound site on a patient. Figure 4A shows a wound site 400 being cleaned and prepared for treatment. Here, the healthy skin surrounding the wound site 400 is preferably cleaned and excess hair removed or shaved. The wound site 400 may also be irrigated with sterile saline solution if necessary. Optionally, a skin protectant may be applied to the skin surrounding the wound site 400. If necessary, a wound packing material, such as foam or gauze, may be placed in the wound site 400. This may be preferable if the wound site 400 is a deeper wound.

After the skin surrounding the wound site 400 is dry, and with reference now to Figure 4B, the wound dressing 100 may be positioned and placed over the wound site 400. Preferably, the wound dressing 100 is placed with the wound contact layer over or in contact with the wound site 400. In some embodiments, an adhesive layer is provided on the lower surface of the wound contact layer, which may in some cases be protected by an optional release layer to be removed prior to placement of the wound dressing 100 over the wound site 400. Preferably, the dressing 100 is positioned such that the fluidic connector 110 is in a raised position with respect to the remainder of the dressing 10 so as to avoid fluid pooling around the port. In some embodiments, the dressing 100 is positioned so that the fluidic connector 110 is not directly overlying the wound, and is level with or at a higher point than the wound. To help ensure adequate sealing for TNP, the edges of the dressing 100 are preferably smoothed over to avoid creases or folds.

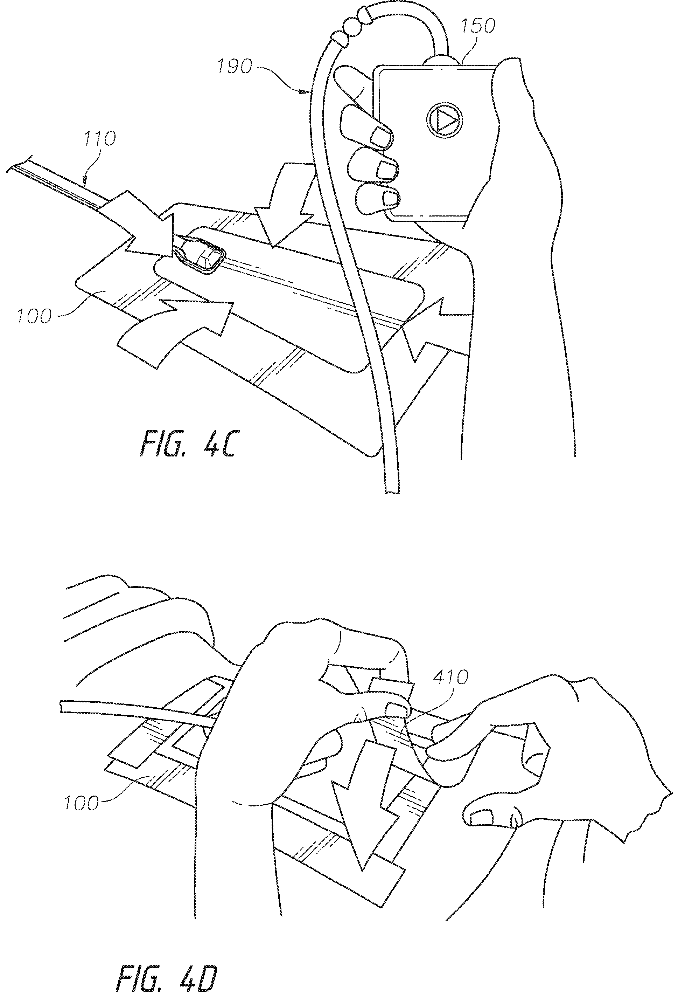

With reference now to Figure 4C, the dressing 10 is connected to the pump 150. The pump 150 is configured to apply negative pressure to the wound site via the dressing 100, and typically through a conduit. In some embodiments, and as described herein, a fluidic connector 110 may be used to join the conduit 190 from the pump to the dressing 100. Where the fluidic connector is adhered to the top layer of the wound dressing, a length of tubing may be coupled at a first end of the fluidic connector such that the tubing, or conduit, extends away from the fluidic connector parallel to the top of the dressing. In some embodiments the conduit may comprise a fluidic connector. It is expressly contemplated that a conduit may be a soft bridge, a hard tube, or any other apparatus which may serve to transport fluid. Upon the application of negative pressure with the pump 150, the dressing 100 may in some embodiments partially collapse and present a wrinkled appearance as a result of the evacuation of some or all of the air underneath the dressing 100. In some embodiments, the pump 150 may be configured to detect if any leaks are present in the dressing 100, such as at the interface between the dressing 100 and the skin surrounding the wound site 400. Should a leak be found, such leak is preferably remedied prior to continuing treatment.

Turning to Figure 4D, additional fixation strips 410 may also be attached around the edges of the dressing 100. Such fixation strips 410 may be advantageous in some situations so as to provide additional sealing against the skin of the patient surrounding the wound site 400. For example, the fixation strips 410 may provide additional sealing for when a patient is more mobile. In some cases, the fixation strips 410 may be used prior to activation of the pump 150, particularly if the dressing 100 is placed over a difficult to reach or contoured area.

Treatment of the wound site 400 preferably continues until the wound has reached a desired level of healing. In some embodiments, it may be desirable to replace the dressing 100 after a certain time period has elapsed, or if the dressing is full of wound fluids. During such changes, the pump 150 may be kept, with just the dressing 100 being changed.

A similar procedure can be followed for application of the wound dressing used without negative pressure. However, the wound dressing does not comprise a port or a fluidic connector and the dressing would not be connected to a negative pressure source as described in Figure 4C.

It can be helpful for clinicians to know what is happening to a wound under the dressing throughout the lifetime of the dressing. Clinicians may want to intervene if the wound shows signs of infection or deterioration and as a result will tend to remove the dressing for inspection of the wound before the dressing has reached its full wear time or capacity or leave the dressing in place too long and end up waiting too long to treat a deteriorated wound. Clinicians rely on visual checks and swabs of the wound taken periodically and the clinician is responsible for deciding how often to check the wound. Therefore, it would be helpful to provide a dressing that incorporates methods of monitoring and visualizing the wound site in real time without removal of the dressing. The treatment of a wound with a wound dressing can be closely monitored by providing information of the wound state through data acquired by the sensors and the ability of that data to trigger an alarm condition. With the alarm, the clinician can be made aware of the condition of the wound without looking and potentially disturbing the wound, adding infection, or missing a change in the condition.

In embodiments according to the invention, the wound dressing utilizes a dressing display to provide information to the user or clinician. In other embodiments not according to the invention the wound dressing may alternatively utilize another audio, visual, or tactile indicator to provide information to the user or clinician. The dressing display can provide visualization to the wound (such, as images of the wound) and information to the user or clinician, in some embodiments, the dressing display can incorporate a flexible OLED (organic light emitting diode) display that is positioned on or underneath the wound backing layer or cover layer and visible by the user or clinician. An OLED flexible display, e-ink display, or equivalent is incorporated into the dressing such that the display faces away from the skin surface to display information to the patient or clinician. The display may be on the surface of the dressing or within the dressing for example under a light transmitting or transparent backing layer of the dressing.

In some embodiments, a scratch resistant film may cover the display to protect it. The film may be separate or form a part of the backing film for the dressing. In some embodiments, the film may also have anti-glareproperties to reduce the problems caused by reflection of light sources. A film may be used that provides immunity to electromagnetic interference (EMI) or radio frequency interference (RFI) (e.g. 9900 - Transparent shielding foil supplied by Holland Shield Systems, such as an optically transparent electrically conductive film). In the case of EMI shielding, the film may be electrically grounded, for example to one or more ground planes of a circuit board, such as a controller circuit board on which a controller is positioned.

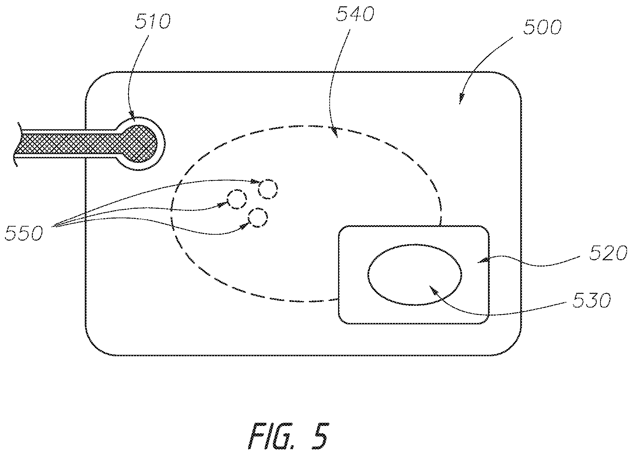

Figure 5 illustrates an embodiment of a wound dressing with a dressing display according to the present invention. The wound dressing 500 with a fluidic connector 510 can be positioned over a wound 540. The wound dressing incorporates a dressing display 520 that faces away from the skin surface to display an image of the wound 530 or other information. As shown in Figure 5, the dressing display 520 is positioned on the outside surface of or underneath the wound backing layer. In some embodiments, the dressing display can be provided above or off to the side or adjacent to the absorbent layer of the dressing. In some embodiments, the absorbent layer can be provided between a high moisture vapor permeable top layer and the wound contact layer, and the dressing display or battery can be provided adjacent to or off to the side of the absorbent layer (either below or above the top layer) so as not to restrict evaporation of fluids from the absorbent layer through the top layer of the dressing.

In some embodiments, controller, battery, or other electrical components may be provided, such as attached to the back of the display, and encapsulated to prevent fluid ingress from the dressing into the electronic module. In some embodiments, the electrical components can be encapsulated or surrounded by a protective casing or coating, for example, a hydrophobic coating. The encapsulation of the electrical components can help assembly of the device as single module that can be inserted into or onto the dressing assembly.

In some embodiments, the dressing can incorporate sensors or electronic components 550 to provide information and status of the wound and assist in wound healing. Sensors 550 may be arranged in/on the dressing for example on the wound contact layer, within the adhesive layer or embedded in materials within the dressing. In some embodiments, the wound contact layer with incorporated or integrated sensors 550 can be provided as a separate layer placed over a wound. Other dressing components can be provided to be placed over the wound contact layer with the integrated sensors. For example, in some embodiments, the wound contact layer with the integrated sensors can be placed in the wound and an integrated wound dressing can be placed over the wound. In some embodiments, the wound contact layer with the integrated sensors can be placed in the wound followed by any other components of the wound dressing and a wound cover layer can be placed over the wound to seal the wound area as described herein. The sensors 550 can be arranged to gather data about the state of the wound, the state of the surrounding tissue, and the ambient conditions of the patient or wound. The sensors can include an array of sensors such as pH, temperature, light, conductivity, impedance, capacitance, or other sensors to detect other characteristics of the wound. The sensors can utilize the host of sensor outputs to provide information about the wound environment and such information can be communicated and displayed on the dressing display. In some embodiments, the sensors can provide information about the blood flow, moister or dryness of the wound, lactate levels, or other characteristics of the wound. In some embodiments, all manner of sensors may be incorporated in the system and they may be configured to measure parameters such as temperature, pH, oxygen, carbon dioxide, conductivity, inductance, lactate, metallomatrix proteases, growth factors, optical absorption and reflectance including at infra red and UV frequencies and fluorescence, infection (level of bacterial burden and types of bacteria), or other characteristics of the wound environment. In some embodiments, the sensors can be used to measure conditions or activities of a patient. For example, the dressing can include sensors that can measure ambient temperature to determine if a patient has showered or accelerometers or gyroscopes to determine if a patient has been walking or been active. Examples of applications where additional disclosure relating to the preceding may be found include U.S. Provisional Application No. 62/336535, titled "SENSOR ENABLED NEGATIVE, PRESSURE WOUND THERAPY APPARATUS," filed on May 13, 2016; and U.S. Provisional Application No. 62/337252, titled "SENSOR ENABLED NEGATIVE PRESSURE WOUND THERAPY APPARATUS," filed on May 16, 2016.

In some embodiments, a source of negative pressure (such as a pump) and some or all other electrical components associated with the topical negative pressure system, such as power source(s), sensor(s), connector(s), user interface component(s) (such as button(s), switch(es), speaker(s), screen(s), etc.) and the like, can be integral with the wound dressing, in some embodiments, the components can be integrated below, within, on top of, or adjacent to the cover layer.

The dressing display can include a controller positioned on or within the dressing. In some embodiments, the controller can be positioned at the center of the dressing. The sensors can feed into controller. In some embodiments, the sensors can communicate with the controller wirelessly through RFID or other wireless communication mechanisms. In some embodiments, the sensors can connect to and communicate with the controller through electrical wiring running through the wound dressing or connected to the wound dressing. The controller is configured to read the data from the sensors and interpret the data for display on the dressing display and transmission to a database and offline/cloud based data processor accessible by the clinician or user. In some embodiments, the display or controller can communicate with other devices through electrical wiring or wirelessly. In some embodiments, the display can transmit and receive wireless data to other hardware or software applications. For example, the display can transmit data, such as optical data, to a remote device, such as a smart phone application. In some embodiments, the wireless data can be transmitted via Bluetooth, infrared, or detection of changes on the display including changes due to pulsed light transmitted from a section or all of the display, or the like. An example of an application where additional disclosure relating to the preceding may be found includes International Application No. PCT/EP2015/080740, titled "NEGATIVE PRESSURE WOUND THERAPY APPARATUS AND METHOD OF OPERATING THE APPARATUS," filed on December 21, 2015, published as WO 2016/107775, published on July 7, 2016. In some embodiments, dressing display or controller can include Global Positioning System (GPS) or another positioning system to provide geolocation or positioning information for the dressing. In some embodiments, GPS can communicate through wireless communication.

In some embodiments, the dressing can be provided with a dressing display incorporated into the wound dressing. In an alternative embodiment, the dressing display can be applied to the wound after the dressing is applied to the patient and secured onto the dressing during use. In some embodiments, the display screen can be square or rectangular in shape and can be about 1 to 2 inches per side. For example, the display screen can be 1 ½ inches square. The dressing display can have a high level of electrical protection, such as against defibrillation, electrostatic discharge (ESD), electrical surgical devices, or the like. For example, isolation coating can be used for electrical protection. Any of the embodiments described herein can utilize one or more electrical protection mechanism described in U.S. Provisional Application No. 62/401727, titled "SYSTEMS AND METHODS FOR APPLYING REDUCED PRESSURE THERAPY," filed on September 29, 2016; U.S. Provisional Application No. 62/401728, titled "SYSTEMS AND METHODS FOR APPLYING REDUCED PRESSURE THERAPY," filed on September 29, 2016; and U.S. Provisional Application No. 62/468258, titled "CONSTRUCTION AND PROTECTION OF COMPONENTS IN NEGATIVE PRESSURE WOUND THERAPY SYSTEMS," filed on March 7, 2017. In some embodiments, the display screen can be a touch screen with user interface components. In some embodiments, the display screen can have an optional touch capability such as projected capacitive touch (PCAP) technology.

In some embodiments, the dressing can be applied with the display preloaded with an image of the wound. An image of the wound can be captured prior to application of the dressing. In some embodiments, the image can be captured with a hyperspectral imaging and other CCD technologies including 3D scan and fluorescence through UV excitation. In some embodiments, the image can be captured with a Smartphone or a Smartphone can received an image of the wound from another device. The Smartphone can then transmit the image to the display on the dressing. The dressing and dressing display can be activated or turned on and the image of the wound can be uploaded to the dressing display. In some embodiments, during operation of the dressing display, the screen of the dressing display can include a power save or power conserving mechanisms. In some embodiments, with the power saving mode or mechanism, during periods of non-use the display screen can be automatically or manually turned off or deactivated to save power or provide privacy for the user. The dressing display can be activated or reactivated from a sleep mode. In some embodiments, the user or clinician can touch the display screen to activate or reactivate the display. In some embodiments, the display screen can be activated or reactivated with an activation mechanism, for example a button or switch, positioned on the dressing or display or the activation mechanism can be remote to the dressing and communicate with the display wirelessly or through electrical wiring.

The dressing in some embodiments can display the wound image on the dressing display. The dressing with the display can be applied to the wound similar to the application described with reference to Figures 4A-4D. In some embodiments, the dressing can be orientated and placed over the wound according to the image on the dressing display. For example, the dressing can be placed over the wound in an orientation that aligns the image with the actual wound. In some embodiments, the display of the dressing can allow for orientation of the image on the OLED display so that the image can be changed or moved once the dressing is placed on the wound. In some embodiments, the camera can be incorporated into the dressing. In such embodiments, it may not be necessary to preload the image prior to application of the dressing as the image wound be captured after application of the dressing to the wound site or during use.

The sensors can be used to take readings over time and gather data relating to the various conditions of the wound. The controller can capture the data and overlay the data on the wound image. In some embodiments, the overlay of the data on the wound image can use color maps, icons, trend lines histograms, or other techniques to visualize or display the characteristics of the wound. Once use of the dressing is discontinued, the dressing is changed and the display dressing can provide a method for the data to be uploaded to a database. In some embodiments, the data can be accessed through data storage mechanisms, for example, cloud storage.

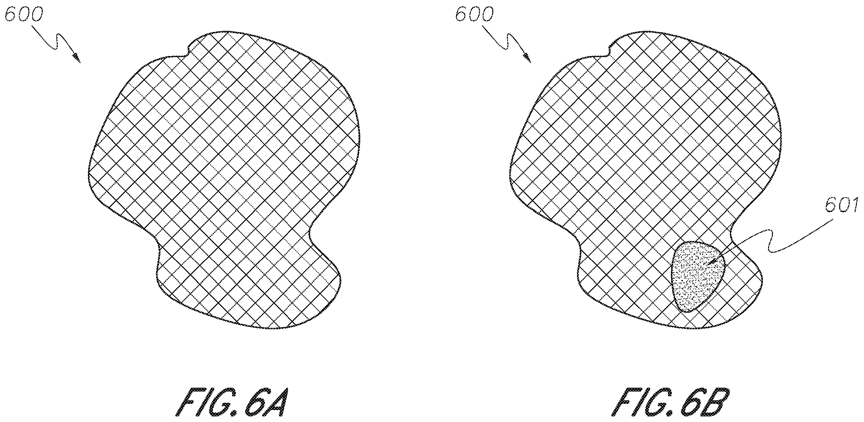

Figures 6A and 6B illustrate a schematic illustration of a wound that can be displayed on the dressing display. Figure 6A illustrates a schematic of a wound 600 with a uniform color or pattern. In some embodiments, a uniform color can indicate that the wound is healing well and there are no abnormalities or issue that require attention or treatment. In some embodiments, if the wound is healing appropriately a color indication can be used, for example, a green color, to indicate that there are no issues. Figure 6B illustrates a schematic of a wound 600 with a suboptimal zone 601 shown in a different pattern. The indication of the suboptimal zone 601 can alert the clinician or user to investigate the wound area or provide treatment. In some embodiments, the suboptimal zone 601 can be shown in a different color than the color of the remainder of the wound area. In some embodiments, the suboptimal zone 601 can be shown on the display with a color indication, for example, a red color. This color indicator can be used to indicate to the clinician that the wound needs to be investigated.

In some embodiments, the dressing display can display a real time schematic or image of the wound with a host of sensor outputs mapped on the wound image. The dressing display can display a black and white or color image of the wound, which can be used, for instance, for clinical diagnostics. In some embodiments, the image can be processed, such as filtered, before being displayed. Such processing can be performed by the controller. The dressing display can display a wound periphery overlay on an original wound shape to show areas that have healed. In some embodiments, the dressing display can display the percent of wound healing or areas not epithelializing. In some embodiments, the dressing display can display sections of the wound via fiber optics, which may facilitate image capture by an external device, such as a device with a camera. In some embodiments, the dressing display can display wound parameters on the dressing display including displaying wound images and physiological parameters of the patient. In some embodiments, data and information relating to the wound parameters and physiological parameters can be gathered by sensors incorporated into the wound as described herein. In some embodiments, information relating to a physiological parameters or vital signs can include pulse rate, blood oxygen saturation (e.g., SPO2), insulin, or any other parameter for providing information on the condition of the patient.

Alarms can be triggered to alert the clinician that the wound condition is sub optimal. In some embodiments, the display can show instructions and provide help text to the user and clinician. In some embodiments, the dressing display can display alerts, alarms, helps, or therapy status from other devices. For example, the dressing display can display alerts, alarms, helps, or therapy status from a negative pressure source or pump in communication with the wound dressing. In some embodiments, the dressing display can communicate with the other devices wirelessly, through a wired connection, or through any other method to transmit this data and information. In some embodiments, the display screen can provide information related to alerts or alarms, such as alarms compliance with one or more medical device operation standards. For example, the display can flash green, yellow, aqua (or other type of blue), and red. In some embodiments, the display screen can indicate a red alert or alarm for exsanguination.

The dressing display can allow for the data relating to the wound and clinical information of the patient to be maintained within the dressing and the dressing display. This can increase patient privacy and alleviate data privacy concerns that arise from physicians carrying smart phones with a patient's medical information.

In some embodiments, the dressing display can support electronic labeling. In some embodiments, the electronic labeling can display information about the system and electronics including operating system information, regulatory and compliance information, manufacturing information including but not limited to lot code or date, and any other information to provide to the user. The information can be preloaded on the dressing display and displayed on the screen by prompt of the user. Regulatory information can vary based on the region and regulatory authority. Therefore, in some embodiments, the dressing display system can utilize GPS or other locating system to provide location information to the system and display only the relevant regulatory or system information for that particular location. In some embodiments, the display can display electronic labels sent from other devices. In some embodiments, the dressing display can display a QR code, display data from other devices, and sensors.

### Other Variations

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

Features, materials, characteristics, or groups described in conjunction with a particular aspect embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. For instance, the various components illustrated in the figures may be implemented as software or firmware on a processor, controller, ASIC, FPGA, or dedicated hardware. Hardware components, such as processors, ASICs, FPGAs, and the like, can include logic circuitry. Furthermore, the features and attributes of the specific embodiments disclosed above may be combined in different ways to form additional embodiments, all of which fall within the scope of the present disclosure.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 0% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

All of the features disclosed in this specification (including any accompanying exhibits, claims, abstract and drawings), or all of the steps of any method or process so disclosed, may be combined in any combination, except combinations where at least some of such features or steps are mutually exclusive. The disclosure is not restricted to the details of any foregoing embodiments.

Various modifications to the implementations described in this disclosure may be readily apparent to those skilled in the art.

## Claims

1. A negative pressure wound therapy apparatus, comprising:
a wound dressing (100; 500) comprising:
a backing layer configured to cover a wound and be positioned over a skin surface surrounding the wound; and
a dressing display (520) positioned on the backing layer and facing away from the skin surface;
wherein the dressing display is configured to be in electrical communication with one or more sensors configured to be positioned within the wound and
wherein the apparatus is **characterized by** a controller configured to capture information about the characteristics of the wound from the one or more sensors, process the information from the one or more sensors and display the captured information on the dressing display.

2. A negative pressure wound therapy apparatus, comprising:
a wound dressing (100; 500) comprising:
a light transmitting or transparent backing layer configured to cover a wound and be positioned over a skin surface surrounding the wound; and
a dressing display (520) positioned underneath the backing layer and facing away from the skin surface;
wherein the dressing display is configured to be in electrical communication with one or more sensors configured to be positioned within the wound and
wherein the apparatus is **characterized by** a controller configured to capture information about the characteristics of the wound from the one or more sensors, process the information from the one or more sensors and display the captured information on the dressing display.

3. The apparatus of claim 1 or claim 2, further comprising the one or more sensors configured to be positioned on or within the wound dressing, the one or more sensors configured to provide information about the characteristics of the wound to the controller.

4. The apparatus of any of the preceding claims, further comprising the controller positioned within or on the wound dressing.

5. The apparatus of any of Claims 1-3, further comprising the controller positioned remote from the wound dressing.

6. The apparatus of any of the preceding claims, wherein the dressing display is a flexible organic light emitting diode display or e-ink display.

7. The apparatus of any of the preceding claims, wherein the sensors are configured to measure one or more of temperature, pH, oxygen, carbon dioxide, conductivity, inductance, lactate, metallomatrix proteases, growth factors, optical absorption, optical reflectance, or infection.

8. The apparatus of any of the preceding claims, wherein the captured information comprises an image of the wound, and the controller is configured to display on the dressing display the image of the wound.

9. The apparatus of any of the preceding claims, further comprising a negative pressure source configured to apply negative pressure to the wound.

10. The apparatus of any of the preceding claims, further comprising a wound contact layer configured to be positioned in contact with the wound.

11. The apparatus of Claim 10, further comprising one or more superabsorbent layers positioned between the wound contact layer and the backing layer.

12. The apparatus of Claim 10 or Claim 11, further comprising a spacer layer between the wound contact layer and the backing layer.

## Patentansprüche

1. Eine Unterdruck-Wundtherapievorrichtung, die Folgendes beinhaltet:
einen Wundverband (100; 500), der Folgendes beinhaltet:
eine Trägerschicht, die konfiguriert ist, um eine Wunde zu bedecken und über einer die Wunde umgebenden Hautoberfläche positioniert zu werden; und
eine Verbandsanzeige (520), die auf der Trägerschicht positioniert ist und von der Hautoberfläche weg zeigt;
wobei die Verbandsanzeige konfiguriert ist, um in elektrischer Kommunikation mit einem oder mehreren Sensoren zu stehen, die konfiguriert sind, um in der Wunde positioniert zu werden, und
wobei die Vorrichtung durch eine Steuereinheit gekennzeichnet ist, die konfiguriert ist, um Informationen über die Eigenschaften der Wunde von dem einen oder den mehreren Sensoren zu erfassen, die Informationen von dem einen oder den mehreren Sensoren zu verarbeiten und die erfassten Informationen auf der Verbandsanzeige anzuzeigen.

2. Eine Unterdruck-Wundtherapievorrichtung, die Folgendes beinhaltet:
einen Wundverband (100; 500), der Folgendes beinhaltet:
eine lichtdurchlässige oder transparente Trägerschicht, die konfiguriert ist, um eine Wunde zu bedecken und über einer die Wunde umgebenden Hautoberfläche positioniert zu werden; und
eine Verbandsanzeige (520), die unter der Trägerschicht positioniert ist und von der Hautoberfläche weg zeigt;
wobei die Verbandsanzeige konfiguriert ist, um in elektrischer Kommunikation mit einem oder mehreren Sensoren zu stehen, die konfiguriert sind, um in der Wunde positioniert zu werden, und
wobei die Vorrichtung durch eine Steuereinheit gekennzeichnet ist, die konfiguriert ist, um Informationen über die Eigenschaften der Wunde von dem einen oder den mehreren Sensoren zu erfassen, die Informationen von dem einen oder den mehreren Sensoren zu verarbeiten und die erfassten Informationen auf der Verbandsanzeige anzuzeigen.

3. Vorrichtung gemäß Anspruch 1 oder Anspruch 2, die ferner einen oder mehrere Sensoren beinhaltet, die konfiguriert sind, um auf oder in dem Wundverband positioniert zu werden, wobei der eine oder die mehreren Sensoren konfiguriert sind, um der Steuereinheit Informationen über die Eigenschaften der Wunde bereitzustellen.

4. Vorrichtung gemäß einem der vorhergehenden Ansprüche, die ferner die Steuereinheit beinhaltet, die in oder auf dem Wundverband positioniert ist.

5. Vorrichtung gemäß einem der Ansprüche 1-3, die ferner die Steuereinheit beinhaltet, die entfernt von dem Wundverband positioniert ist.

6. Vorrichtung gemäß einem der vorhergehenden Ansprüche, wobei die Verbandsanzeige eine flexible organische Leuchtdiodenanzeige oder eine E-Ink-Anzeige ist.

7. Vorrichtung gemäß einem der vorhergehenden Ansprüche, wobei die Sensoren konfiguriert sind, um eines oder mehrere von Folgendem zu messen: Temperatur, pH-Wert, Sauerstoff, Kohlendioxid, Leitfähigkeit, Induktivität, Laktat, Metallmatrixproteasen, Wachstumsfaktoren, optische Absorption, optische Reflexion oder Infektion.

8. Vorrichtung gemäß einem der vorhergehenden Ansprüche, wobei die erfassten Informationen ein Bild der Wunde beinhalten und die Steuereinheit konfiguriert ist, um das Bild der Wunde auf der Verbandsanzeige anzuzeigen.

9. Vorrichtung gemäß einem der vorhergehenden Ansprüche, die ferner eine Unterdruckquelle beinhaltet, die konfiguriert ist, um Unterdruck auf die Wunde auszuüben.

10. Vorrichtung gemäß einem der vorhergehenden Ansprüche, die ferner eine Wundkontaktschicht beinhaltet, die konfiguriert ist, um in Kontakt mit der Wunde positioniert zu werden.

11. Vorrichtung gemäß Anspruch 10, die ferner eine oder mehrere superabsorbierende Schichten beinhaltet, die zwischen der Wundkontaktschicht und der Trägerschicht positioniert sind.

12. Vorrichtung gemäß Anspruch 10 oder Anspruch 11, die ferner eine Abstandsschicht zwischen der Wundkontaktschicht und der Trägerschicht beinhaltet.

## Revendications

1. Un appareil de thérapie des plaies par pression négative, comprenant :
un pansement pour plaie (100 ; 500) comprenant :
une couche dorsale configurée pour recouvrir une plaie et être positionnée par-dessus une surface de peau entourant la plaie ; et
une unité d'affichage de pansement (520) positionnée sur la couche dorsale et orientée à l'opposé de la surface de peau ;
dans lequel l'unité d'affichage de pansement est configurée pour être en communication électrique avec un ou plusieurs capteurs configurés pour être positionnés au sein de la plaie et
l'appareil étant **caractérisé par** un organe de commande configuré pour capturer des informations sur les caractéristiques de la plaie provenant des un ou plusieurs capteurs,
traiter les informations provenant des un ou plusieurs capteurs et afficher les informations capturées sur l'unité d'affichage de pansement.

2. Un appareil de thérapie des plaies par pression négative, comprenant :
un pansement pour plaie (100 ; 500) comprenant :
une couche dorsale transmettant la lumière ou transparente configurée pour recouvrir une plaie et être positionnée par-dessus une surface de peau entourant la plaie ; et
une unité d'affichage de pansement (520) positionnée en dessous de la couche dorsale et orientée à l'opposé de la surface de peau ;
dans lequel l'unité d'affichage de pansement est configurée pour être en communication électrique avec un ou plusieurs capteurs configurés pour être positionnés au sein de la plaie et
l'appareil étant **caractérisé par** un organe de commande configuré pour capturer des informations sur les caractéristiques de la plaie provenant des un ou plusieurs capteurs, traiter les informations provenant des un ou plusieurs capteurs et afficher les informations capturées sur l'unité d'affichage de pansement.

3. L'appareil de la revendication 1 ou de la revendication 2, comprenant en outre les un ou plusieurs capteurs configurés pour être positionnés sur ou au sein du pansement pour plaie, les un ou plusieurs capteurs étant configurés pour fournir des informations sur les caractéristiques de la plaie à l'organe de commande.

4. L'appareil de n'importe lesquelles des revendications précédentes, comprenant en outre l'organe de commande positionné au sein du ou sur le pansement pour plaie.

5. L'appareil de n'importe lesquelles de revendications 1 à 3, comprenant en outre l'organe de commande positionné à distance du pansement pour plaie.

6. L'appareil de n'importe lesquelles des revendications précédentes, dans lequel l'unité d'affichage de pansement est une unité d'affichage à diodes électroluminescentes organiques ou une unité d'affichage à encre électronique souple.

7. L'appareil de n'importe lesquelles des revendications précédentes, dans lequel les capteurs sont configurés pour mesurer un ou plusieurs éléments parmi la température, le pH, l'oxygène, le dioxyde de carbone, la conductivité, l'inductance, le lactate, des métalloprotéases matricielles, des facteurs de croissance, l'absorption optique, la réflectance optique, ou une infection.

8. L'appareil de n'importe lesquelles des revendications précédentes, dans lequel les informations capturées comprennent une image de la plaie, et l'organe de commande est configuré pour afficher sur l'unité d'affichage de pansement l'image de la plaie.

9. L'appareil de n'importe lesquelles des revendications précédentes, comprenant en outre une source de pression négative configurée pour appliquer une pression négative à la plaie.

10. L'appareil de n'importe lesquelles des revendications précédentes, comprenant en outre une couche de contact avec la plaie configurée pour être positionnée en contact avec la plaie.

11. L'appareil de la revendication 10, comprenant en outre une ou plusieurs couches superabsorbantes positionnées entre la couche de contact avec la plaie et la couche dorsale.

12. L'appareil de la revendication 10 ou de la revendication 11, comprenant en outre une couche d'espaceur entre la couche de contact avec la plaie et la couche dorsale.
